# FASCICULE DE BREVET EUROPEEN

(11) **EP 0 881 902 B1**
(45) Date de publication et mention de la délivrance du brevet: **18.12.2002**
(21) Numéro de dépôt: 97901665.6
(22) Date de dépôt: 24.01.1997
(51) Int. Cl.: A61K 31/695

(54) **ASSOCIATIONS DE LIPIDES PEROXYDES ET DE COMPOSES ORGANOSILICIQUES, COMPOSITIONS COSMETIQUES ET DERMATOLOGIQUES LES CONTENANT ET LEURS APPLICATIONS NOTAMMENT POUR LE TRAITEMENT DE L'ALOPECIE**
KOSMETISCHE ODER DERMATOLOGISCHE ZUBEREITUNGEN, ENTHALTEND PEROXIDLIPIDE UND ORGANOSILIZIUMVERBINDUNGEN UND VERWENDUNG DERSELBEN
COMBINATIONS OF PEROXIDE LIPIDS AND ORGANOSILICON COMPOUNDS, COSMETIC AND DERMATOLOGICAL COMPOSITIONS CONTAINING SAME, AND USES THEREOF, IN PARTICULAR FOR TREATING ALOPECIA

(30) Priorité: 26.01.1996 FR 9600922
(43) Date de publication de la demande: 09.12.1998
(73) Titulaire: LABORATOIRES CARILENE, 78362 Montesson Cédex (FR)
(72) Inventeur: DESJONQUERES, Stéphane, F-78600 Maisons Laffitte (FR)
(74) Mandataire: Giraud, Françoise
(86) Numéro de dépôt international: FR9700141
(87) Numéro de publication internationale: WO97026892

(56) Documents cités:
- DE-A- 4 419 783
- FR-A- 2 645 863

## Description

La présente invention concerne de nouvelles associations de lipides peroxydés et de composés organosiliciques, les compositions cosmétiques et dermatologiques les contenant ainsi que leurs applications, notamment pour le traitement de l'alopécie.

La chute des cheveux encore appelée alopécie est un problème tout particulièrement répandu dans l'ensemble de la population, notamment dans la population masculine mais également dans la population féminine.

De nombreuses études ont été consacrées à ce problème. En ce qui concerne les alopécies féminines diffuses, on citera notamment la mise au point sur les alopécies féminines diffuses réalisées par P. Reygagne parue dans bedc, 1993, 1, 7, 327-338. De cette étude, il ressort que les alopécies diffuses font actuellement l'objet de trois types de traitements :
- des traitements généraux tels que l'association de vitamine B5 et de vitamine H ou de vitamine B8,
- le minoxidil prescrit par voie locale qui provoque une repousse modérée dans 30 % des cas mais dont l'effet positif cesse de se faire sentir dans les 3 à 6 mois suivant l'arrêt des applications,
- des traitements endocrinologiques dans le cas des alopécies andro-génétiques.

D'une façon générale, l'alopécie androgénétique est la plus fréquente des alopécies et, sans doute, celle où la demande thérapeutique est la plus forte. Toutefois, les traitements anti-androgénétiques per os ayant une efficacité certaine chez la femme ne sont pas réalisables chez l'homme et aucun traitement anti-androgène local n'a pu trouver une efficacité durable. Le seul traitement local ayant pu démontrer dans plusieurs études contrôlées une efficacité certaine chez l'homme est le minoxidil en application locale biquotidienne. Les premières études faites entre 1982 et 1986 étaient relativement optimistes avec 18 à 53 % de repousse. Une telle étude est relatée dans : Androgenic alopecia : clinical aspects and treatment, in "Hair and Hair Diseases" C.E. ORPHANOS, R HAPPLE (Eds), Springer-Verlag, Berlin Heidelberg 1990, PP 485-527. En fait, les repousses cosmétologiquement acceptables sont rares, comme relaté par RUSHTON D.H., VUGER W.P., COTTERIL P.C., KINGSLEY P., JAMES K.C. dans "Quantitative assessment of 2 % topical Minoxidil in the treatment of male pattern baldness", Clin Exp Dermatol, 1989, 14, 40-6. De plus, OLSEN E.A., WEINER M.S. AMARA LA., DELONG E.R., ont signalé dans l'article intitulé "Five-year follow-up of men with androgenetic alopecia treated with topical Minoxidil" dans J. Am Acad Dermatol 1990, 22, 643-6, une lente reprise de la chute au-delà de 1 an de traitement même si celui-ci est poursuivi. Par ailleurs, une diffusion systémique limite l'augmentation de la concentration locale du minoxidil et les préparations actuellement commercialisées sont toutes dosées à 2 % de minoxidil.

On connaît différents lipides peroxydés, notamment obtenus par peroxydation d'huile végétale naturelle. On citera, en particulier, les brevets suivants BSM N°2 330 M, EP-A-293 535, FR-A-2 591 112, EP-A-225 831, EP-A-225 832, EP-A-225 833, EP-A-226 506, FR-A-2 461 744, FR-A-2 539 142 et EP-A-117 962 qui concernent soit la préparation de tels lipides peroxydés soit leurs applications dans différents domaines, en particulier dans le traitement de certaines affections dans le domaine de la rhumatologie ou de la traumatologie, ou encore en tant que produit cicatrisant.

Des lipides peroxydés de ce type ont également été utilisés selon le brevet européen EP-A-0 480 983 pour le traitement par application locale des insuffisances circulatoires.

On trouve dans la littérature de nombreuses références concernant l'activité du silicium organique sur la stimulation de la biosynthèse du collagène ou de l'élastine, dans la reconstitution du tissu conjonctif lésé. Il ressort à travers les travaux expérimentaux relatés dans les différentes publications, qu'en particulier, le silicium organique provoque un retour à la normale de la paroi artérielle dégradée par l'attaque d'un atérome artificiellement provoqué.

Plus précisément, dans J. Med. Esth et Chir. Derm., volume XII, 47, septembre 1985, pages 187-190, F. MARCHI-LIPSKI et F. DANIEL ont fait une synthèse bibliographique concemant l'intérêt thérapeutique du silicium dans le vieillissement du tissu conjonctif. Il ressort de cette étude que le silicium présente un intérêt tout particulier dans le maintien de l'intégrité du tissu conjonctif et que les dérivés organosiliciés permettent de lutter efficacement contre le vieillissement du tissu conjonctif sous toutes ses formes.

Il ressort également de cet article qu'il existe des dérivés organiques du silicium solubles qui peuvent être utilisés selon diverses voies d'administration et notamment par application locale.

Parmi les nombreuses applications thérapeutiques citées dans cet article, on relève l'application pour améliorer la croissance et la solidité des cheveux.

Dans le chapitre consacré à l'activité biologique des silatranes dans Topics in Current Chemistry, vol. 84, pages 77-135, 1979, Michail G. Voronkov a mis en évidence l'intérêt de ces silatranes dans le traitement de l'alopécie.

Par ailleurs, le brevet français FR-2-645 863 décrit des composés de silicium sous forme de complexes moléculaires d'un silanol préparés par réaction d'un sel alcalin ou ammonium d'un silanol avec un acide en présence d'une zéolite. Ces complexes de silanols peuvent être, en autres, utilisés pour améliorer la repousse des cheveux.

C'est ce type de complexes qui est utilisé pour la préparation du produit vendu sous le nom commercial M 44 par les Laboratoires Carilène.

Un tel produit destiné à arrêter la chute des cheveux agit du fait de la présence de silicium organique comme :
. tonifiant des parois vasculaires, conduisant à l'amélioration de la vascularisation papillaire,
. régulateur des métabolismes cellulaires favorisant le passage en phase anagène du cycle pilaire,
. stimulant de la synthèse de collagène et d'élastine permettant la restructuration de la tige pilaire,
. séborégulateur, conduisant à la diminution de l'excès de sébum.

Poursuivant ses recherches en vue d'améliorer les performances des produits destinés au traitement de l'alopécie, l'inventeur de la présente invention a constaté que des associations d'huiles peroxydées et de dérivés organiques du silicium, en particulier de silanols conduisaient à un renforcement considérable des activités des deux produits permettant des performances parfaitement inattendues dans le traitement de l'alopécie, en particulier de l'alopécie de type androgénétique.

En effet, ces associations permettent d'obtenir des résultats au moins équivalents à ceux obtenus avec le minoxidil tout en évitant les inconvénients de ce dernier produit, à savoir le défaut de rémanence de l'effet observé lors de l'arrêt du traitement. Par ailleurs, un autre avantage non négligeable de ces associations par rapport au minoxidil est qu'elles ne provoquent aucune augmentation de la séborrhée au cours du traitement et ne nécessitent qu'une seule application quotidienne et non deux.

Une étude contrôlée randomisée de l'efficacité de ces associations a permis de démontrer sans ambiguïté l'efficacité de ces associations. L'efficacité des associations a été comparée avec celle de minoxidil, seul produit de référence actuel dans le traitement de l'alopécie androgénétique. La méthode expérimentale pour réaliser cette comparaison a été celle des phototrichogrammes, la seule reconnue aujourd'hui comme étant parfaitement fiable. Le protocole et le résultat de cette étude sont relatés dans les exemples du présent mémoire.

Ainsi donc, l'invention concerne selon un premier aspect, à titre de produit industriel nouveau, des associations des deux types de produits chimiques, à savoir des huiles peroxydées et des composés organiques du silicium, en particulier des dérivés de silanols.

Elle concerne également selon un deuxième aspect des compositions cosmétiques ainsi que des compositions pharmaceutiques, notamment dermatologiques, contenant ces associations à titre de principe actif.

Elle concerne également, selon un autre de ses aspects essentiels, l'utilisation de ces associations et compositions dans le traitement de l'alopécie.

Elle concerne, selon un autre aspect, d'autres applications de ces associations et compositions dans lesquelles le même effet de potentialisation de l'effet d'une des deux substances actives par l'autre a pu être observé. C'est, en particulier, ce qui est observé pour le traitement des effets du vieillissement de la peau, la cicatrisation et la régénération tissulaire ainsi que dans le traitement de la cellulite.

Plus précisément, l'invention concerne selon une de ses caractéristiques essentielles une association, notamment utile comme principe actif d'une composition cosmétique ou pharmaceutique, notamment dermatologique, caractérisée en ce qu'elle contient 1 à 6 parties en poids de lipides peroxydés pour 0,01 à 0,1 partie en poids calculé par rapport au silicium organique d'un dérivé organosilicié biologiquement actif.

Selon un mode particulièrement avantageux, ces associations sont sous forme d'émulsions stables.

Les lipides peraxydés susceptibles d'être utilisés conformément à l'invention peuvent être de nature chimique très variée mais présentent avantageusement un taux de peroxydation compris entre 30 et 500 milli-équivalents par kilo, de préférence entre 50 et 300 milli-équivalents par kilo, de préférence encore entre 50 et 150 milli-équivalents par kilo.

Selon un mode de réalisation particulier, ces lipides présentent avantageusement une teneur en glycérides oxydés comprise entre 5 et 40 %.

Conformément à l'invention, on utilise de préférence comme lipides peroxydés au moins un peroxyde obtenu par peroxydation de lipides d'origine végétale, par exemple sous forme d'au moins une huile végétale naturelle. De préférence, ces huiles sont choisies parmi l'huile d'amande douce, l'huile de noisette, l'huile d'arachide, l'huile de maïs, l'huile de pépins de raisin, l'huile de sésame et l'huile de carthame.

Selon un mode de réalisation particulier de l'invention, on utilise des lipides peroxydés constitués principalement ou majoritairement de triglycérides répondant à la formule générale : 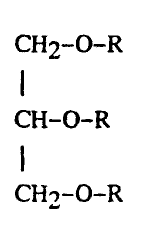 dans laquelle les radicaux R sont majoritairement représentés par les acides octa-décanoïques et octdécanoïques peroxydés.

Les dérivés organosiliciés biologiquement actifs utiles selon l'invention peuvent être tout dérivé organique du silicium soluble dans l'eau et connu pour son activité biologique.

Il peut s'agir de dérivés solubles d'organosilanol, d'organosilanediol ou d'organosilanetriol libres ou condensés. Ces dérivés solubles sont avantageusement sous forme de sels ou de complexes des organosilanols correspondants, désignés ci-dessous par silanol par mesure de simplication.

Ces dérivés seront avantageusement choisis parmi les dérivés solubles des organosilanols de formule [RₙSi(OH)₄₋ₙ)x, dans laquelle O < x ≤ 4, les n groupements R sont identiques ou différents et représentent indépendamment de l'hydrogène ou un groupement alkyle ou aralkyle et n est compris entre 1 et 3.

La nature des différents groupements R est telle que lesdits organosilanols correspondent à des dérivés solubles et non toxiques.

A titre d'exemple de tels dérivés solubles de silanols, on citera : le monométhylsilanetriol de potassium, le diméthylsilisalycilate perhydrolysé, le mannuronate de monométhylsilanetriol, le hyaluronate de diméthylsilanol, l'ascorbyl méthylsilanol pectinate, le méthylsilanol aspartate hydroxyprolinate.

D'une façon générale, on pourra utiliser comme dérivés de silanol tous les organosilanols solubles dans l'eau, donc biologiquement actifs et assimilables.

A titre d'autres exemples de dérivés de silanol utilisables pour préparer les associations utiles selon l'invention, on citera également les composés du silicium décrits dans le brevet français FR 2 645 863. Il s'agit, plus précisément, de composés du silicium sous forme de complexe moléculaire d'un silanol de formule générale : 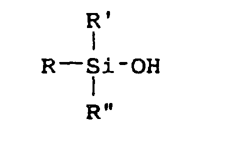 où R est un groupement alkyle ou aryle et R' et R" sont des groupements alkyle, aryle ou OH, et d'un dérivé alcalin ou ammonium d'un acide.

De tels produits sont obtenus en particulier à partir d'un sel alcalin ou ammonium d'un silanol que l'on combine avec un acide en présence d'une zéolite permettant une réaction complète des deux produits.

Comme cela ressort clairement des exemples qui suivent, l'intérêt des associations selon l'invention est qu'elles peuvent être utilisées comme principe actif de compositions cosmétiques ou pharmaceutiques, notamment dermatologiques, en conférant à ces compositions non seulement les avantages cumulés des deux types de principes actifs qui les composent mais également une véritable synergie de ces activités.

Cette synergie s'avère particulièrement intéressante dans le cas du traitement de l'alopécie, en particulier de l'alopécie androgénétique.

Les associations de l'invention permettent, du fait de la présence des triesters de glycérol oxydés, d'agir sur le rétablissement quasi-immédiat des flux sanguins artériels au niveau du follicule pileux et, par conséquent, de provoquer un afflux sanguin qui peut jouer rapidement sur l'arrêt de la chute excessive des cheveux et même permettre, à terme, la repousse de velus.

Parallèlement à cette action, le silicium organique soluble contenu dans l'association intervient à la fois pour véhiculer le premier principe actif et pour rendre aux artères lésées du follicule leur motricité, ce qui assure un maintien à long terme des flux sanguins normaux, même après l'arrêt du traitement, grâce à la régénération du tissu conjonctif artériel.

Ainsi, il se crée une synergie des deux activités thérapeutiques liées à l'action d'accélérateur des flux sanguins et à l'action cicatrisante par activation du renouvellement cellulaire dû à la présence des triesters de glycérols oxydés et à l'action de catalyseur de la biosynthèse du tissu conjonctif dû à la présence des dérivés de silicium qui conduisent à la restructuration de la paroi des veines, des artères et des capillaires.

Les deux principes actifs constituant les associations de l'invention ont donc ainsi une action conjuguée qui conduit à une meilleure irrigation au niveau du follicule pileux en même temps qu'à une régénération du système nourricier des cheveux.

Ainsi donc, selon un de ses aspects essentiels, la présente invention concerne des compositions cosmétiques ou pharmaceutiques, notamment dermatologiques contenant à titre de principe actif une association telle que décrite précédemment.

Ces compositions contiennent avantageusement de 1 à 6 % en poids de lipides peroxydés et de 0,01 à 0,1 % en poids, exprimé par rapport au silicium organique d'un dérivé organosilicié biologiquement actif, en particulier d'un dérivé soluble d'un silanol, et un véhicule cosmétiquement ou pharmaceutiquement acceptable.

Ces compositions sont utilisables dans tous les domaines où l'on recherche une potentialisation de l'activité des lipides peroxydés par celle des dérivés du silicium.

Cet effet a particulièrement été observé dans le cas du traitement de l'alopécie, en particulier de l'alopécie androgénétique, comme cela ressort des exemples.

Toutefois, l'intérêt des associations n'est pas limité à ce type de traitement puisqu'elles peuvent être utilisées dans tout type de traitements cosmétiques ou pharmaceutiques, notamment dermatologiques où l'on cherche à soigner ou traiter la peau, le cuir chevelu ou les cheveux, en vue en particulier de lutter contre les effets du vieillissement, à améliorer la cicatrisation et la régénération tissulaire ainsi qu'à lutter contre la chute des cheveux et à améliorer leur repousse.

Selon ces procédés, on applique sur la partie du corps à traiter une quantité cosmétiquement ou pharmaceutiquement active d'une composition telle que décrite précédemment.

En conséquence, si les associations selon l'invention s'avèrent particulièrement efficaces pour la préparation de compositions pharmaceutiques, notamment dermatologiques, destinées à être appliquées sur le cuir chevelu et/ou les cheveux en vue du traitement de l'alopécie, notamment de l'alopécie androgénétique, elles s'avèrent également très utiles dans différents traitements tels que le traitement de la cellulite, le soin ou le traitement de la peau en vue de lutter contre son vieillissement, ainsi que tout traitement destiné à améliorer la cicatrisation ou la régénération tissulaire.

Les compositions selon l'invention pourront se trouver sous toutes formes galéniques utilisables en fonction de l'effet recherché et de la zone du corps à traiter. Il pourra, en particulier, s'agir de lotions, de gels, de crèmes, de shampooings, ces formes galéniques étant destinées au cuir chevelu ou à l'application sur le visage ou le corps.

Les exemples suivants sont donnés à titre purement illustratif de l'invention.

### Exemples

Dans les exemples ci-dessous, les concentrations, sauf indication contraire, sont donnés en pourcentages en poids.

### Exemple 1

| Formule d'une crème destinée au soin du corps ou du visage | |
|---|---|
| Triesters de glycérols oxydés | 10 |
| Monométhylsilanetriol de potassium | 0,5 |
| Excipients, conservateurs, parfums | qsp 100 |

Cette crème est appliquée en légers massages 1 ou 2 fois par jour en traitement de 3 mois minimum.

Cette crème est particulièrement utile pour améliorer le renouvellement cellulaire, lutter contre la cellulite et les vergetures. Elle améliore, en outre, la cicatrisation et agit comme anti-inflammatoire et a un effet antalgique et apaisant.

### Exemple 2

| Formule d'une lotion destinée au soin du corps ou du visage | |
|---|---|
| Triesters de glycérols oxydés | 3 |
| Monométhylsilanetriol de potassium | 0,1 |
| Alcool cosmétique 99° | 15 |
| Eau permutée, parfums, conservateurs, | qsp 100 |

Cette lotion est appliquée à l'aide d'un coton à raison de quelques gouttes de lotion, le soir, sur le corps ou le visage. L'application est renouvelée pendant 3 mois minimum .

Cette lotion est particulièrement utile pour améliorer le renouvellement cellulaire, lutter contre la cellulite et les vergetures. Elle améliore, en outre, la cicatrisation et agit comme anti-inflammatoire et a un effet antalgique et apaisant.

### Exemple 3

### Lotion capillaire

On prépare une composition présentant la composition ci-dessous, en pourcentages en poids.

| | |
|---|---|
| Citrate de potassium de monométhylsilanetriol | 1 |
| Hydrolysat de protéines | 0,5 |
| Hydrolysat de kératine | 0,5 |
| Complexe d'aminoacide | 0,1 |
| Huile de maïs peroxydé (Epaline 100®) | 3 |
| Alcool absolu pour cosmétologie | 10 |
| Essence de gardiénia | 0,05 |
| Excipient, conservateur et eau déminéralisée | qsp 100 |

Cette lotion est appliquée sur le cuir chevelu, raie par raie, à raison de quelques gouttes, le soir de préférence. L'application est renouvelée pendant 3 moil minimum.

### Exemple 4

### Test clinique d'efficacité anti-alopécique

L'efficacité de la composition de l'exemple 3 contre la chute et la séborrhée a été testée, en comparaison avec minoxodil sur une population de 60 hommes de 18 à 65 ans en pleine évolution d'alopécie androgénétique, présentant une alopécie de type androgénétique au stade III à VI selon la classification de Hamilton.

L'essai comparatif a été réalisé dans les conditions suivantes sur une période de 9 mois. Les patients ont été répartis en deux groupes homogènes et de même importance.

### a) Protocole de test

### a - 1 : Bilan initial

Chacun des patients a été soumis à un bilan initial (à J0) comportant :
1 - La mesure du diamètre de la zone alopécique sur le sommet du crâne.
2 - Le remplissage d'un questionnaire.
3 - La réalisation d'un phototrichogramme pré-thérapeutique:
   Celui-ci nécessite dans un premier temps le rasage et le tatouage semi-permanent de la zone à photographier. Ce tatouage est réalisé à l'aide d'aiguilles stériles à 3 branches à usage unique. La zone est choisie en avant du vertex dans une zone alopécique présentant un pourcentage de cheveux télogènes a priori supérieur à 20 %. Sur une surface de 2 cm² environ, les cheveux sont coupés ras à J0 avec l'aide d'un ciseau courbe. Deux points de tatouage semi-permanent permettent ensuite de repérer un carré fictif de 7 x 7 mm au sein duquel seront comptabilisés les cheveux. Ces deux points de tatouage correspondront à deux repères fixes du système de prise de vue. Il faut veiller à ce que les cheveux aient tous une même orientation parallèle à l'intérieur du carré fictif de 7 x 7 mm.
   Après cette opération, une macrophotographie est effectuée à J0 dans des conditions de distance, d'éclairage et de grossissement (x 3) standardisées et parfaitement reproductibles. Une seconde photographie comparative est effectuée à J0 + 2 jours. La comparaison des deux photographies permet de différencier les cheveux en phase anagène qui ont poussé, des cheveux en phase télogène qui n'ont pas poussé.
   Cette technique permet donc d'étudier :
      - la densité de cheveux au cm²,
      - le nombre de cheveux anagènes (A),
      - le nombre de cheveux télogène (T),
      - le rapport A/T,
      - le pourcentage des cheveux anagènes (A%)
      - la vitesse de croissance des cheveux,
      - leur diamètre (D).
   Le phototrichogramme est répété après les 3 mois de la première période de traitement (M₃), une 3ème fois 3 mois plus tard, à l'issue de la deuxième période de traitement (M₆).
4 - Prélèvements des cheveux
   Une mèche de cheveux est prélevée sur la zone à raser pour le phototrichogramme. Cette mèche sera liée à la base, stockée dans un tube numéroté et permet de mesurer ultérieurement les diamètres des tiges pilaires.

### a - 2 : Traitement

Après réalisation du premier phototrichogramme chaque patient se voit remettre un coffret comportant les 3 premiers mois de traitement (13 semaines). Ces coffrets ont numérotés de 1 à 60 et les numéros sont attribués à chaque patient selon son ordre d'arrivée dans l'étude. Chaque coffret comporte soit 3 flacons de Minoxidil, soit 3 flacons de lotion de l'exemple 3. Chaque flacon de Minoxidil et chaque flacon de lotion selon l'invention porte une étiquette autocollante rappelant le numéro du coffret auquel il appartient.

L'essai étant randomisé, les numéros des coffrets (30 coffrets Minoxidil et 30 coffrets de lotion de l'exemple 3) sont attribués grâce à une table de randomisation équilibrée tous les 4 patients. Lors de la période de traitement d'une durée de 13 semaines, chacun patient utilise soit 1 ml de Minoxidil en application locale tous les jours matin et soir, soit 3 ml de lotion de l'exemple 3 en application locale une seule fois par jour le soir.

A la fin de la première période de traitement (temps noté M3), après la deuxième visite de surveillance et la réalisation du 2ème phototrichogramme, un 2ème coffret numéroté de traitement a été remis à chaque patient. Il comporte encore soit le produit de l'exemple 3, soit le Minoxidil. La deuxième période de traitement dure également 3 mois (13 semaines). A la fin de cette période (temps noté M6), un 3ème phototrichogramme est effectué. Enfin, un 4ème phototrichogramme a été effectué sur les différents patients après arrêt complet du traitement pendant 13 semaines (temps noté M9).

Au cours des traitements, les traitements suivants sont autorisés : Shampooing : Ils sont libres. Les patients peuvent se laver les cheveux tous les jours s'ils le souhaitent, avec le shampoing de leur choix. Le mieux est qu'ils poursuivent leur shampooing habituel si celui-ci leur convenait. Colorations, décolorations et permanentes : Elles sont autorisées. Il faudra simplement éviter de les faire pendant les périodes de réalisation des phototrichogrammes.

Par contre, ne sont pas autorisés :

Tous les traitements locaux ou généraux pouvant avoir une incidence sur la pousse ou la chute des cheveux sont interdits toute la durée de l'étude, et au cours des 3 mois précédent l'inclusion. Sont en particulier interdits les prostaglandines, les agents rubéfiants, tous les vasodilatateurs, les anti-androgènes et tous les traitements hormonaux locaux.

### b) Résultats

L'étude a montré pour les numérations de cheveux anagènes, télogènes, cheveux totaux ainsi que les rapports anagène/télogène entre les mois M₀ et M₆, une équivalence entre les deux produits, puisque les très légères différences que l'on peut remarquer, dont certaines en faveur du produit de l'exemple 3, ne sont pas statistiquement significatives.

La projection clinique de ces numérations conduit le clinicien a conclure à une activité anti-chute réelle des deux produits ainsi qu'a une certaine activité sur la repousse.

L'étude statistique menée à partir des résultats des phototrichogrammes conduit à conclure à l'équivalence entre les deux produits testés en ce qui concerne les rapports anagènes (A) sur télogènes (T). Ces résultats sont clairement exprimés dans le tableau 1 ci-dessous :

**TABLEAU 1**

| Evolution du rapport entre M6 et M0 | | | | |
|---|---|---|---|---|
| | | Groupe de traitement | | Total |
| | | Lotion de l'exemp 3 | Minoxidil | |
| Différence M6/M0 : A/T | N | 32,00 | 28,00 | 60,00 |
| | Données manquantes | 1,00 | 0,00 | 1,00 |
| | Moyenne | 0,0 | -0,5 | -0,2 |
| | Ecart-type | 2,3 | 2,9 | 2,6 |
| | Minimum | -4,75 | -9,13 | -9,13 |
| | Maximum | 7,33 | 6,14 | 7,33 |

où il apparaît qu'aucune différence n'a été décelée entre les deux groupes de traitement concernant l'évolution du rapport du nombre de cheveux anagènes sur le nombre de télogènes entre M6 et M0.

Par ailleurs, aucune différence significative n'a été décelée pour cette même évolution entre M3 et M0 (p = 0,057).

Par ailleurs, le tableau 2 ci-dessous montre qu'aucune différence n'a été observée pour les deux produits concemant la différence des pourcentages de cheveux anagènes entre M6 et M0.

**TABLEAU 2**

| Pourcentage de cheveux anagènes-différence M6-M0 | | | | |
|---|---|---|---|---|
| | | Groupe de traitement | | Total |
| | | Lotion de l'exemple 3 | Minoxidil | |
| Différence M6/M0: % Anagènes | N | 32,00 | 28,00 | 60,00 |
| | Données manquantes | 1,00 | 0,00 | 1,00 |
| | Moyenne | -0,4 | -3,2 | -1,7 |
| | Ecart-type | 11,0 | 12,8 | 11,9 |
| | Minimum | -32,54 | -39,80 | -39,80 |
| | Maximum | 23,66 | 22,40 | 23,66 |

Par ailleurs, aucune différence n'a été décelée entre les deux groupes de traitement entre M3 et M0.

Par contre, le tableau 3 ci-dessous montre que le nombre total de cheveux dans la cible dépend du traitement suivi.

**TABLEAU 3**

| Nombre total de cheveux dans la cible-différence M6-0 | | | | |
|---|---|---|---|---|
| | | Groupe de traitement | | Total |
| | | Lotion de l'exemple 3 | Minoxidil | |
| Différence M6/M0 : Total | N | 32,00 | 28,00 | 60,00 |
| | Données manquantes | 1,00 | 0,00 | 1,00 |
| | Moyenne | -2,9 | 6,4 | 1,4 |
| | Ecart-type | 9,4 | 9,4 | 10,5 |
| | Minimum | -22,00 | -18,00 | -22,00 |
| | Maximum | 22,00 | 28,00 | 28,00 |

Une différence significative (p < 0,001) a été décelée entre les deux groupes de traitement concernant l'évolution du nombre total de cheveux dans la cible entre M6 et M0.

A titre indicatif, il y avait également une différence significative (p = 0,001) entre M3 et M0. Cette différence peut être expliquée par l'augmentation du nombre de cheveux télogènes dans le groupe minoxidil. Il faut noter que ce type de cheveux (télogènes) est amené à tomber dans les 3 mois.

Une différence très nette est également observée en ce qui concerne le nombre de cheveux télogènes au bout de 6 mois de traitement. Cette différence ressort clairement du tableau 4 ci-dessous.

**TABLEAU 4**

| Nombre de cheveux télogènes-différence M6-M0 | | | | |
|---|---|---|---|---|
| | | Groupe de traitement | | Total |
| | | Lotion de l'exemple 3 | Minoxidil | |
| Différence M6/M0 : T | N | 32,00 | 28,00 | 60,00 |
| | Données manquantes | 1,00 | 0,00 | 1,00 |
| | Moyenne | -0,8 | 3,4 | 1,2 |
| | Ecart-type | 6,2 | 8,1 | 7,4 |
| | Minimum | -16,00 | -12,00 | -16,00 |
| | Maximum | 10,00 | 29,00 | 29,00 |

Une différence significative (p = 0,025) a été décelée entre les deux groupes de traitement concernant l'évolution du nombre de cheveux télogènes entre M6 et M0.

A titre indicatif, il n'y avait pas de différence pour cette évolution entre M3 et M0.

Des résultats de l'étude statistique relatés dans les tableaux ci-dessus, il ressort toutefois une meilleure qualité des cheveux après traitement de 6 mois par la lotion de l'invention par rapport à celle observée après traitement par minoxidil puisque, même si le minoxidil favorise davantage la repousse des cheveux, les cheveux télogènes se trouvent en pourcentage plus élevé.

D'un point de vue clinique, on doit prendre en compte les améliorations importantes apportées par le produit de l'exemple 3, en comparaison avec le minoxidil :
- Une seule application par jour (au lieu de 2)
- Un produit non gras qui n'impose pas le shampoing après usage
- Une absence de séborrhée (le minoxidil stimule la production de sébum par les glandes sébacées)
- Un cheveu dont le diamètre a augmenté par rapport au groupe minoxidil
- Une approche globale des résultats à M6 a amené le clinicien à préjuger d'une action à long terme, ce qui devrait permettre l'arrêt du traitement une fois de bons résultats obtenus, alors que le minoxidil exige un traitement à vie, puisque le maintien des résultats obtenus est lié à un usage ininterrompu. Cette activité "retard" a été confirmée par les résultats à M9
- Un faible pourcentage d'alcool.

Les résultats de cette étude comparative sur 9 mois figurent dans le tableau 5 ci-dessous en ce qui concerne le produit minoxidil de référence pour une application bi-quotidienne de ce produit pendant les 6 premiers mois de l'étude et dans le tableau 6 ci-dessous pour le produit de l'exemple 3, appliqué 1 fois par jour, pendant les 6 premiers mois du traitement, les traitements étant arrêtés dans les deux cas au bout de 6 mois.

**TABLEAU 5**

| Produit de référence | | | | |
|---|---|---|---|---|
| | M0 | M3 | M6 | M9 |
| A | 45,89 | 59,33 | 49,43 | 42,71 |
| T | 14,29 | 11,83 | 17,57 | 19,10 |
| A/T | 3,21 | 5,01 | 2,82 | 2,23 |
| A% | 76,55 | 83,93 | 74,03 | 69,49 |
| N* | 61,40 | 70,77 | 67,00 | 61,81 |
| D (µm) | 70,00 | 73,71 | 77,18 | 77,76 |

| | | | | |
|---|---|---|---|---|
| N* : Nombre de cheveux dans la cible | | | | |

**TABLEAU 6**

| Produit de l'exemple 3 | | | | |
|---|---|---|---|---|
| | M0 | M3 | M6 | M9 |
| A | 47,24 | 51,45 | 46,73 | 44,65 |
| T | 15,64 | 12,21 | 13,23 | 15,96 |
| A/T | 3,02 | 4,21 | 3,53 | 2,79 |
| A% | 75,07 | 80,59 | 76,68 | 73,61 |
| N * | 62,68 | 63,67 | 60,27 | 60,92 |
| D (µm) | 74,85 | 79,68 | 80,31 | 80,27 |

Le protocole expérimental de l'étude clinique comparative avec minoxidil, prévoyait de prolonger l'étude pendant trois mois après l'arrêt du traitement.

Les paramètres de croissance - anagènes, pourcentage d'anagènes, nombre de cheveux totaux - sont restés d'une remarquable stabilité dans le groupe traité par le produit de l'exemple 3, par opposition à une reprise rapide de la pathologie dans le groupe traité par minoxidil.

En conséquence, le schéma thérapeutique conseillé avec le produit de l'exemple 3 consiste à profiter de cet effet "retard" et à prévoir des périodes d'arrêt de traitement de trois mois, après les premiers trois mois de traitement initial. On envisagera donc un traitement de trois mois suivi de trois mois d'arrêt du traitement puis reprise du traitement pendant trois mois, etc.

Ceci représente évidemment un avantage important pour l'utilisateur du produit, qu'il s'agisse de qualité de vie ou de prix de traitement.

## Revendications

1. Association constituée d'un mélange d'un dérivé organosilicié biologiquement actif et de lipides peroxydés et contenant de 1 à 6 parties en poids de lipides peroxydés pour 0,01 à 0,1 partie en poids calculé par rapport au silicium organique dudit dérivé organosilicié biologiquement actif.

2. Association selon la revendication 1, **caractérisée en ce qu'**elle est sous la forme d'une émulsion stable.

3. Association selon la revendication 1 ou 2, **caractérisée en ce que** lesdits lipides peroxydés présentent un taux de peroxydation compris entre 30 et 500 milli-équivalents par kilo, de préférence entre 50 et 300 milli-équivalents par kilo, de préférence encore entre 50 et 150 milli-équivalents par kilo.

4. Association selon l'une des revendications 1 à 3, **caractérisée en ce que** les lipides peroxydés précités sont obtenus par peroxydation de lipides d'origine végétale, par exemple sous forme d'au moins une huile végétale naturelle.

5. Association selon la revendication 4, **caractérisée en ce que** l'huile naturelle est choisie dans le groupe consistant de l'huile d'amande douce, de l'huile de noisette, de l'huile d'arachide, de l'huile de maïs, de l'huile de pépins de raisin, de l'huile de sésame et de l'huile de carthame.

6. Association selon l'une des revendications 1 à 5, **caractérisée en ce que** les lipides peroxydés précités présentent avantageusement une teneur en glycérides oxydés comprise entre 5 et 40 %.

7. Association selon l'une des revendications 1 à 6, **caractérisée en ce que** ledit dérivé organosilicié est un dérivé soluble dans l'eau d'un organosilanol d'un organosilanediol ou d'un organosilanetriol.

8. Association selon la revendication 7, **caractérisée en ce que** ledit dérivé soluble dans l'eau est un dérivé d'un silanol de formule [RₙSi(OH)₄₋ₙ)x, dans laquelle O < x ≤ 4, les n groupements R sont identiques ou différents et représentent indépendamment de l'hydrogène ou un groupement alkyle ou aralkyle et n est compris entre 1 et 3.

9. Composition cosmétique ou pharmaceutique, notamment dermatologique, **caractérisée en ce qu'**elle contient à titre de principe actif une association selon l'une des revendications 1 à 8.

10. Composition selon la revendication 9, **caractérisée en ce qu'**elle contient de 1 à 6 % en poids de lipides peroxydés et de 0,01 à 0,1 % en poids, exprimé par rapport au silicium organique, d'un dérivé organosilicié biologiquement actif et un véhicule cosmétiquement ou pharmaceutiquement acceptable.

11. Procédé de traitement cosmétique de la peau, du cuir chevelu ou des cheveux, destiné à lutter contre les effets du vieillissement, à améliorer la cicatrisation et la régénération tissulaire ainsi qu'à lutter contre la chute des cheveux et améliorer leur repousse, **caractérisé en ce qu'**il consiste à appliquer sur la partie du corps à traiter une quantité cosmétiquement active d'une composition selon l'une des revendications 9 ou 10.

12. Utilisation d'une association telle que définie dans l'une des revendications 1 à 8 comme principe actif pour la fabrication d'une composition pharmaceutique, notamment dermatologique destinée à être appliquée sur le cuir chevelu et/ou les cheveux en vue du traitement de l'alopécie, notamment de l'alopécie androgénétique.

## Patentansprüche

1. Assoziation, bestehend aus einer Mischung aus einem biologisch aktiven Organosilicium-Derivat und peroxidierten Lipiden, die 1 bis 6 Gew.-Teile peroxidierte Lipide auf 0,01 bis 0,1 Gew.-Teil des biologisch aktiven Organosilicium-Derivats, bezogen auf das organische Silicium, enthält.

2. Assoziation nach Anspruch 1, **dadurch gekennzeichnet, dass** sie in Form einer stabilen Emulsion vorliegt.

3. Assoziation nach Anspruch 1 oder 2, **dadurch gekennzeichnet, dass** die peroxidierten Lipide einen Peroxidationsgrad zwischen 30 und 500 Milliäquivalenten pro kg, vorzugsweise zwischen 50 und 300 Milliäquivalenten pro kg, besonders bevorzugt zwischen 50 und 150 Milliäquivalenten pro kg, aufweisen.

4. Assoziation nach einem der Ansprüche 1 bis 3, **dadurch gekennzeichnet, dass** die peroxidierten Lipide erhalten werden durch Peroxidation von Lipiden pflanzlichen Ursprungs, beispielsweise in Form mindestens eines natürlichen Pflanzenöls.

5. Assoziation nach Anspruch 4, **dadurch gekennzeichnet, dass** das natürliche Öl ausgewählt wird aus der Gruppe, die besteht aus süßem Mandelöl, Nussöl, Erdnussöl, Maisöl, Traubenkernöl, Sesamöl und Safloröl.

6. Assoziation nach einem der Ansprüche 1 bis 5, **dadurch gekennzeichnet, dass** die vorgenannten peroxidierten Lipide zweckmäßig einen Gehalt an oxidierten Glyceriden zwischen 5 und 40 % aufweisen.

7. Assoziation nach einem der Ansprüche 1 bis 6, **dadurch gekennzeichnet, dass** das Organosilicium-Derivat ein in Wasser lösliches Derivat eines Organosilanols, eines Organosilandiols oder eines Organosilantriols ist.

8. Assoziation nach Anspruch 7, **dadurch gekennzeichnet, dass** das in Wasser lösliche Derivat ein Derivat eines Silanols der Formel [RₙSi(OH)₄₋ₙ]ₓ ist, worin O < x ≤ 4 ist, die n Gruppen R identisch oder verschieden sind und unabhängig voneinander für Wasserstoff oder eine Alkyl- oder Aralkylgruppe stehen und n zwischen 1 und 3 liegt.

9. Kosmetische oder pharmazeutische Zusammensetzung, insbesondere dermatologische Zusammensetzung, **dadurch gekennzeichnet, dass** sie als Wirkstoff eine Assoziation nach einem der Ansprüche 1 bis 8 enthält.

10. Zusammensetzung nach Anspruch 9, **dadurch gekennzeichnet, dass** sie 1 bis 6 Gew.-% peroxidierte Lipide und 0,01 bis 0,1 Gew.-%, bezogen das organische Silicium, eines biologisch aktiven Organosilicium-Derivats und ein kosmetisch oder pharmazeutisch verträgliches Vehiculum enthält.

11. Verfahren zur kosmetischen Behandlung der Haut, der behaarten Schädelhaut oder der Haare, zur Bekämpfung von Alterserscheinungen, zur Verbesserung der Vernarbung und der Regenerierung von Gewebe sowie zur Bekämpfung des Haarausfalls und zur Verbesserung des erneuten Haarwuchses, **dadurch gekennzeichnet, dass** man auf den zu behandelnden Körperteil eine kosmetisch aktive Menge einer Zusammensetzung nach einem der Ansprüche 9 oder 10 aufbringt.

12. Verwendung einer Assoziation, wie sie in einem der Ansprüche 1 bis 8 definiert ist, als Wirkstoff zur Herstellung einer pharmazeutischen Zusammensetzung, insbesondere einer dermatologischen Zusammensetzung, die bestimmt ist für den Auftrag auf die behaarte Schädelhaut und/oder die Haare zur Behandlung des Haarausfalls, insbesondere des Haarausfalls beim Männern.

## Claims

1. A combination constituted of a biologically active organosilicon compound and of peroxidised lipids and containing 1 to 6 parts by weight of peroxidised lipids per 0.01 to 0.1 part by weight based on the organic silicon of said biologically active organosilicon compound.

2. The combination according to claim 1, **characterised in that** it is in the form of a stable emulsion.

3. The combination according to claim 1 or 2, **characterised in that** said peroxidised lipids have a peroxidation rate between 30 and 500 milli-equivalents per kilo, preferably between 50 and 300 milli-equivalents per kilo, preferably still between 50 and 150 milli-equivalents per kilo.

4. The combination according to one of claims 1 to 3, **characterised in that** the above-mentioned peroxidised lipids are obtained by peroxidation of lipids of plant origin, for example in the form of at least one natural vegetable oil.

5. The combination according to claim 4, **characterised in that** the natural oil is selected from the group consisting of sweet almond oil, hazelnut oil, peanut oil, maize oil, grape seed oil, sesame oil and oil of safflower.

6. The combination according to one of claims 1 to 5, **characterised in that** the above-mentioned peroxidised lipids advantageously have a glyceride oxides content between 5 and 40 %.

7. The combination according to one of claims 1 to 6, **characterised in that** said organosilicon derivative is a water-soluble organosilanol, organosilanediol or organosilanetriol derivative.

8. The combination according to claim 7, **characterised in that** said water-soluble derivative is a silanol derivative of formula [RₙSi(OH)₄₋ₙ]x, in which O < x ≤ 4, the n groups R are identical or different and represent independently hydrogen or an alkyl or aralkyl group and n is between 1 and 3.

9. A cosmetic or pharmaceutical composition, notably dermatological composition, **characterised in that** it contains as active principle a combination according to one of claims 1 to 8.

10. The composition according to claim 9, **characterised in that** it contains from 1 to 6 % by of peroxidised lipids and from 0.01 to 0.1 % by weight, based on the organic silicon, of a biologically active organosilicon derivative and a cosmetically or pharmaceutically acceptable vehicle.

11. A method of cosmetic treatment of the skin, the scalp or the hair, for fighting against the effects of ageing, for improving healing and tissue regeneration as well as for fighting against hair loss and improving hair re-growth, **characterised in that** it consists in applying onto the part of the body to be treated a cosmetically active amount of a composition according to one of claims 9 or 10.

12. Use of a combination as defined in one of claims 1 to 8 as active principle for the preparation of a pharmaceutical composition, notably dermatological composition for application onto the scalp and/or the hair with the view of treating alopecia, notably androgenetic alopecia.
